# FASCICULE DE BREVET EUROPEEN

(11) **EP 1 551 821 B1**
(45) Date de publication et mention de la délivrance du brevet: **17.05.2006**
(21) Numéro de dépôt: 03808761.5
(22) Date de dépôt: 16.10.2003
(51) Int. Cl.: C07D 307/86, C07D 307/94, C07D 405/12, C07D 213/48, C07C 47/548, C07C 47/55, A61K 31/343, A61P 25/18

(54) **DERIVES DE 3-(CYCLOPENTEN-1-YL)-BENZYL- OU 3-(CYCLOPENTEN-1-YL)-HETEROARYLMETHYL-AMINES ET LEUR UTILISATION A TITRE DE MEDICAMENTS POUR LE TRAITEMENT DE LA SCHIZOPHRENIE**
3-(CYCLOPENTEN-1-YL)-BENZYL- ODER 3-(CYCLOPENTEN-1-YL)HETEROARYLMETHYLAMINDERIVATE UND DEREN VERWENDUNG ALS ARZNEIMITTEL ZUR BEHANDLUNG VON SCHIZOPHRENIE
3-(CYCLOPENTEN-1-YL)-BENZYL- OR 3-(CYCLOPENTEN-1-YL)-HETEROARYLMETHYL-AMINE DERIVATIVES AND USE THEREOF AS MEDICINES FOR TREATING SCHIZOPHRENIA

(30) Priorité: 16.10.2002 FR 0212854
(43) Date de publication de la demande: 13.07.2005
(73) Titulaire: PIERRE FABRE MEDICAMENT, 92100 Boulogne-Billancourt (FR)
(72) Inventeur: VACHER, Bernard, F-81100 Castres (FR); CUISIAT, Stéphane, 81100 Castres (FR); KOEK, Wouter, San Antonio, TX 78255 (US); COLPAERT, Francis, F-81700 Puylaurens (FR)
(74) Mandataire: Ahner, Francis
(86) Numéro de dépôt international: PCT/FR2003/003053
(87) Numéro de publication internationale: WO 2004/035561

(56) Documents cités:
- WO-A-00/58282
- WO-A-99/51575
- WO-A-99/58527
- CHEMICAL ABSTRACTS, vol. 119, no. 17, 25 octobre 1993 (1993-10-25) Columbus, Ohio, US; abstract no. 180657, YANUSAGA T. ET AL: "Preparation of (hetero)aryloxyalkylamine derivatives having selective affinity to serotonin (5-HT 1A)receptors" XP002245145 & JP 05 125024 A (YAMANOUCHI PHARMA CO LTD) 21 mai 1993 (1993-05-21) cité dans la demande

## Description

La schizophrénie est une maladie mentale grave et invalidante qui affecte plus de 50 millions de personnes dans le monde (Sciences 2002, 296(5598), 692-5). Les mécanismes qui sous-tendent les psychoses schizophréniques sont complexes. Il semble néanmoins établit qu'un disfonctionnement de la transmission dopaminergique soit impliqué dans leurs symptomatologies (Nature 1988, 336, 783-87 ; Pharmacol. Rev. 2001, 53(1), 119-33). En effet, les antagonistes des récepteurs dopaminergiques centraux, en particulier des récepteurs du sous-type D₂ (e.g., halopéridol, chlorpromazine...) constituent une approche classique et cliniquement efficace du traitement des psychoses schizophréniques, en particulier des symptômes dit positifs ou productifs (Nature 1976, 261, 717-19). Les composés possédant un tel mécanisme d'action induisent cependant des effets secondaires, non corrélés à l'action thérapeutique, tels que des symptômes de type parkinsonien (Pharmacotherapy 1996, 16, 160), des dyskinésies tardives, des désordres endocriniens... (Drug Metab. Dispos. 1997, 25(6), 675-84).

Une autre classe d'agents antipsychotiques, dit atypiques, a été introduite plus récemment (ID 2002, 3(7), 1073-80). En terme d'avantages thérapeutiques, l'intérêt de à ces nouveaux agents par rapport aux agents conventionnels réside dans :
- une plus faible propension à provoquer des effets secondaires d'ordre neurologique, en particulier les effets extra-pyramidaux (J. Clin. Psychiatry 2000, 61(S3), 10-5) ;
- une activité anti-déficitaire accrue (CNS Drugs 2002, 16(4), 249-61) ;
- une efficacité plus grande dans certaines formes de schizophrénie réfractaires (CNS Drugs 2002, 16(7), 473-84).

Ces composés atypiques (e.g., clozapine, rispéridone, olanzapine...) agissent, en général, à la fois comme des antagonistes dopaminergique et sérotoninergique, en particulier au niveau des récepteurs du type 5-HT₂ (Psychopharmacol. Bull. 1989, 25, 390-92 ; Psychopharmacoly 1993, 112, S40-S54). Chacun de ces médicaments possède, néanmoins, un profil d'affinité différent, non seulement au niveau des sous-types de récepteurs dopaminergique et sérotoninergique mais aussi au niveau des récepteurs muscarinique, adrénergique et histaminique. Ainsi, il ne semble pas se dégager de profil d'affinité caractéristique d'un statut « atypique ».

Il ressort, néanmoins, de plusieurs études cliniques (Br. Med. J. 2000, 321, 1360-61 et 1371-76) qu'en général :
- les agents atypiques ne sont pas plus efficaces que les agents dit conventionnels, au moins sur le plan des symptômes positifs (ou productifs) ; l'impact sur les syndromes déficitaires (ou négatifs) étant plus difficile à objectiver en clinique humaine ;
- les agents atypiques présentent une meilleure tolérance neurologique que les agents conventionnels mais induisent par ailleurs des effets secondaires qui leur sont propres (e.g., prise de poids, diabète, troubles sexuels, toxicité hématologique et/ou cardiaque...) ; certains de ces effets secondaires étant aussi graves que les effets extra-pyramidaux parfois associés aux traitements par les agents conventionnels (Br. Med. J. 2002, 325, 243-5).

Globalement, les approches thérapeutiques existantes pour le traitement des psychoses schizophréniques ne sont donc pas entièrement satisfaisantes (J. Med. Chem. 2001, 44(4) 477-501). La découverte de nouveaux traitements plus efficaces et mieux tolérés est de ce fait fortement souhaitable.

Il a été montré chez l'animal que les agonistes 5-HT1_{A} sont, entres autres, capables de s'opposer à la catalepsie (J. Neural Transm. 1991, 83(1-2), 43-53 ; J. Pharmacol. Exp. Ther. 1993, 265(1), 207-17 ; Eur. J. Pharmacol. 1998, 356, 189-92) et d'atténuer l'augmentation du taux plasmatique de prolactine (J. Pharmacol. Exp. Ther. 1989, 249, 23641) induit par des antagonistes D₂. Les agonistes 5-HT1_{A} ont aussi la capacité d'augmenter la libération de dopamine et d'acétylcholine dans le cortex préfrontal (Brain Res. 2002, 939, 34-42) ; propriétés que n'intègrent pas les agents conventionnels et qui sont supposées contribuer à l'activité anti-déficitaire des agents dit « atypiques » (J. Psychopharmacol. 2001, 15(1) 37-46). Enfin, les effets anxiolytiques et anti-dépresseurs des agonistes 5-HT1_{A} constituent un avantage lors du traitement des psychoses schizophréniques. L'association dans un même médicament d'une activité du type antagoniste des récepteurs D₂ et agoniste des récepteurs du sous-type 5-HT1_{A} est donc, en théorie, fortement désirable puisqu'elle confèrerait à la fois un spectre d'activité plus large (e.g., symptômes positifs, activité anti-déficitaire, anti-dépressive...) et une meilleure tolérance que les agents conventionnels (e.g., effets extra-pyramidaux) et que la plupart des agents atypiques. Compte tenu de l'intérêt thérapeutique potentiel représenté par une combinaison antagoniste D₂ et agoniste 5-HT1_{A} de nombreux composés ayant un tel profil sont décrits dans la littérature (J. Pharmacol. Exp. Ther. 2000, 295(3), 853-61). On peut citer, à titre d'exemple, des dérivés d'arylpipérazine (e.g., Bioorg. Med. Chem. Lett. 2001, 11, 2345-49 ; J. Med. Chem. 2001, 44, 186-97 ; Biorg. Med. Chem. Lett. 1999, 9, 1679-82 ; Pharmazie 2001, 56, 803-07; J. Med. Chem. 1998, 41, 2010-18 ; Pharmazie, 1998, 53, 438-41 ; Arzneim-Forsch. 1997, 47, 239-43 ; Med. Chem. Res. 1997, 7, 76-86 ; Pharmazie 1997, 52, 423-8 ; J. Med. Chem. 1994, 37, 99-104 ; DE 10043659 ; WO 0216354 ; WO 9811068 ; WO 9703067 ; J. Med. Chem. 1992, 35, 552-58 ; J. Med. Chem. 1995, 38, 1498-20 ; WO 9955672 ; US 6,310,066 ; J. Med. Chem. 1998, 41, 760-71 et WO 09711070 ; Bioorg. Med. Chem. Lett. 2001, 11, 2345-49 ; Drug of the Future 2001, 26, 128-32 ; Exp. Opin. Ther. Patents 1998, 8, 737-40 et EP 900792 ; EP 770066 ; WO 9736893 ; WO 9711070 et J. Med. Chem. 1998, 41, 760-71 ; WO 9818797 ; WO 0168063) ; des dérivés d'aminotétraline (e.g., Bioorg. Med. Chem. Lett. 1999, 9, 1583-86 ; Biorg. Med. Chem. Lett. 1999, 7, 1263-71 et 2541-48 ; Bioorg. Med. Chem. Lett. 1999, 7, 2541-48 ; J. Med. Chem. 1993, 36, 1053-68) ; des dérivés de benzodioxane (e.g., EP 707007 ; WO 0172741 ; WO 9840386 ; WO 9829415 ; WO 9723485 ; WO 9507274 et J. Med Chem. 1999, 42, 3342-55 ; WO 9717343 ; EP 669331) ; des dérivés d'aryloxyéthylamine (e.g., WO 0198293 ; WO 9808817 ; US 5958965 ; WO 9951576).

Néanmoins, malgré la profusion de composés décrits comme antagoniste D₂ et agoniste 5-HT1_{A}, un seul reste disponible cliniquement (i.e., némonapride : RN 75272-39-8) et trois sont rapportés en développement actif dans l'indication neuroleptique (PJB Publications Ltd. 2002) i.e., SSR-181507 (Sanofi-Synthelabo), bifeprunox et SLV-313 (Solvay). Le contraste entre le nombre de candidats et le nombre de composés en clinique illustre, entre autres, les difficultés à obtenir des effets additionnels à partir de la sollicitation concomitante de deux systèmes distincts au moyen d'une seule entité chimique. A ce titre, la demanderesse a découvert que plusieurs composés dérivés de (3-cyclopenten-1-yl-[benzyl ou pyrid-3-ylmethyl])-(2-aryloxy-ethyl)-amine interagissent sélectivement avec les récepteurs dopaminergiques des sous-types D₂/D₃ et sérotoninergiques du sous-type 5-HT1_{A} aux niveau desquels ils se comportent, respectivement, comme des antagonistes et des agonistes. Comme les agents conventionnels et à la différence des composés dit « atypiques », les composés de l'invention présentent l'avantage, in vivo, de bloquer efficacement les récepteurs du type D₂ et donc d'être potentiellement actifs dans le traitement des symptômes productifs des schizophrénies. Cependant, contrairement aux agents conventionnels et à certains agents atypiques, les composés de l'invention n'occasionnent pas de catalepsie chez l'animal même à des doses très supérieures aux doses pharmacologiques. L'induction de catalepsie chez l'animal est connue comme étant représentative des effets extra-pyramidaux qui se manifestent chez l'homme. Le profil d'activité des composés de l'invention et donc, à ce titre, tout à fait singulier. En tant que tel, les composés de l'invention sont donc potentiellement utiles pour le traitement des psychoses schizophréniques pour lesquelles il existe un besoin thérapeutique important.

L'état de la technique le plus proche est représenté par les composés décrits dans les brevets JP 05255302 et JP 05125024 de formule : 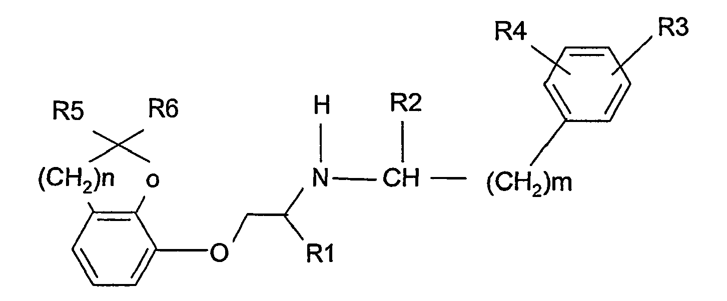 dans laquelle :
R1, R2, R5 et R6 peuvent être un atome d'hydrogène ou un groupe alcoyle simple ;
R3 et R4 représentent, entre autres, un atome d'hydrogène ou un groupe alcoyle simple ;
m est compris entre 1 et 5 ;
n est compris entre 1 et 4.

Les composés en question sont revendiqués comme étant des ligands sélectifs des récepteurs 5-HT1_{A} utiles pour le traitement des désordres affectant le système nerveux central.

Les brevets US 6,121,307 et WO 9951575 revendiquent des N-[(aryloxy)éthyl]indoylalkylamines de formule : 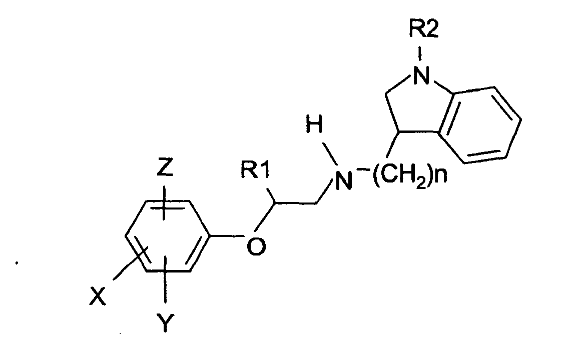 dans laquelle :
R1 peut-être un atome d'hydrogène ;
X et Y peuvent former un hétérocycle de type furanyl ou dihydrofuranyl ;
n est compris entre 2 et 5 ;
comme agents actifs au niveau du système sérotoninergique, en particulier sur les récepteurs 5-HT1_{A}, utiles dans le traitement des dépressions.

L'illustration de l'état de la technique peut en outre être complétée par la citation de la demande internationale WO 99/58527 qui décrit des dérivés de benzofurane et plus particulièrement de pipéridin - et de pipérazinyl - alkylbenzofurane utiles dans le traitement d'affections du coeur ou du système nerveux central. Il ne s'agit cependant nullement d'amines bifonctionnalisées par des groupements de type aryle.

Les composés de l'invention diffèrent donc de dérivés de l'art antérieur à la fois par leur mécanisme d'action et leur formule chimique. Par exemple, le fragment [3-cyclopenten-1-yl-benzylamino] n'apparaît que dans des dérivés du type 4-(1-cyclopenten-1-yl)-2-[(dialkylamino)méthyl]-phénols utilisés comme agents complexant (Izv. Vyssh. Uchebn. Zaved., Khim. Tekhnol. 1980, 23(4), 406-11).

L'intérêt majeur des composés de l'invention se situe donc dans leur action complémentaire, voire dans certains cas synergique, au niveau des systèmes sérotoninergique et dopaminergique. En effet, nous montrons in vivo que la courbe dose-effets (i.e., normalisation des stéréotypies due à l'activation des récepteurs dopaminergiques) de certains composés de l'invention est déplacée vers la droite en présence de l'antagoniste 5-HT1_{A} sélectif WAY-100635 (RN 162760-96-5). Inversement, ces mêmes produits deviennent fortement cataleptigènes en présence de WAY-100635. Cette synergie d'activité, inattendue au vue des résultats des composés de l'art antérieur revendiquant un mécanisme d'action mixte, similaire (Psychopharmacol. 1999, 144(1), 20-29) ouvre des perspectives thérapeutiques nouvelles en clinique humaine dans un domaine pour lequel les médicaments existants ne sont pas entièrement satisfaisants.

Plus spécifiquement, la présente invention a pour objet les nouveaux dérivés du type [(benzofuranyl-7-oxy)-éthyl]-[(cyclopenten-1-yl)-{aryl ou hétéroaryl}méthyl]-amine qui, sous forme de base, répondent à la formule générale (1) : 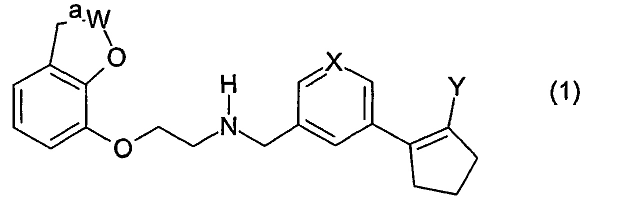 dans laquelle :
- **(a)** représente une liaison simple ou une liaison double ;
- **W** représente un groupe CH, CH₂, CHCH₃, CCH₃, C(CH₃)₂, un groupe C(CH₂)₂ (i.e., un atome de carbone portant deux groupes méthylène liés entre eux de manière à former un motif spiro-cyclopropane) ou C(CH₂)₃ (i.e., un atome de carbone portant deux groupes méthylène liés à un autre groupe méthylène de manière à former un motif spiro-cyclobutane) avec la réserve, toutefois, que lorsque **(a)** est une liaison double alors **W** représente exclusivement un groupe CH ou CCH₃ et que, lorsque **(a)** est une liaison simple, alors **W** représente exclusivement un groupe CH₂, CHCH₃, C(CH₃)₂, C(CH₂)₂ ou C(CH₂)₃ ;
- **X** est un atome de carbone portant un atome d'hydrogène (CH) ou un atome d'azote ;
- **Y** est un atome d'hydrogène ou un atome de fluor ;
leurs sels d'addition et éventuellement les hydrates des sels d'addition avec les acides minéraux ou les acides organiques pharmaceutiquement acceptables ainsi que leurs formes tautomères, les énantiomères purs et les mélanges d'énantiomères racémique ou non.

Certains composés de l'invention comportent un atome de carbone asymétrique dans leur structure. De ce fait, ils existent sous la forme d'énantiomères. L'invention concerne aussi bien chaque énantiomère pur, c'est à dire associé avec moins de 5% de l'autre énantiomère, que leur mélange en toutes proportions. Les composés de l'invention peuvent donc intervenir en tant qu'énantiomères purs ou mélanges racémiques ou non-racémiques.

L'invention s'étend enfin au procédé de préparation des dérivés de formule générale (1).

Les dérivés de formule générale (1) peuvent être obtenus par le procédé décrit dans le schéma A.

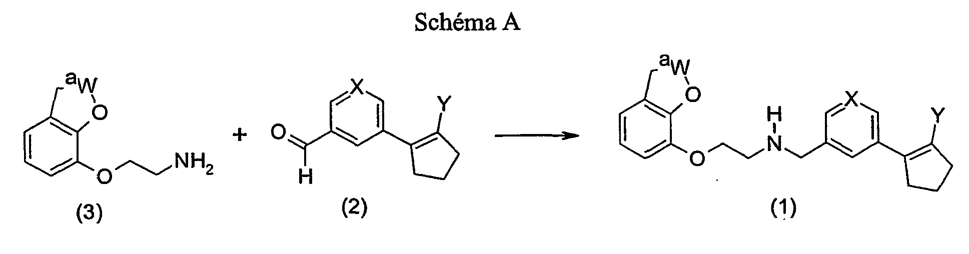

### Schéma A

Le composé de formule (1) est préparé par une réaction classique d'amination réductrice entre l'aldéhyde de formule (2), dans lequel **X** et **Y** ont la même signification que précédemment, et l'amine primaire de formule (3), dans lequel (a) et **W** ont la même signification que précédemment. L'expression "une réaction classique d'amination réductrice" signifie que l'aldéhyde de formule (2) et l'amine primaire de formule (3) sont mis en réaction dans un solvant approprié et que le mélange des réactifs (2) et (3) est ensuite soumis à l'agent réducteur selon une méthode bien connue du chimiste organicien.

Les composés de formule (1) sont purifiés suivant une ou plusieurs méthodes choisies parmi la cristallisation et/ou les techniques de chromatographie en phase liquide. Ils peuvent être ensuite, si on le désire, salifiés au moyen d'un acide pharmaceutiquement acceptable.

La préparation des aldéhydes de formule (2) dépend de la nature des groupes **X** et **Y**.

Ainsi, la préparation de l'aldéhyde (2a) dans lequel **X** représente un atome de carbone portant un atome d'hydrogène (CH) et **Y** est un atome d'hydrogène est décrite dans le schéma B.

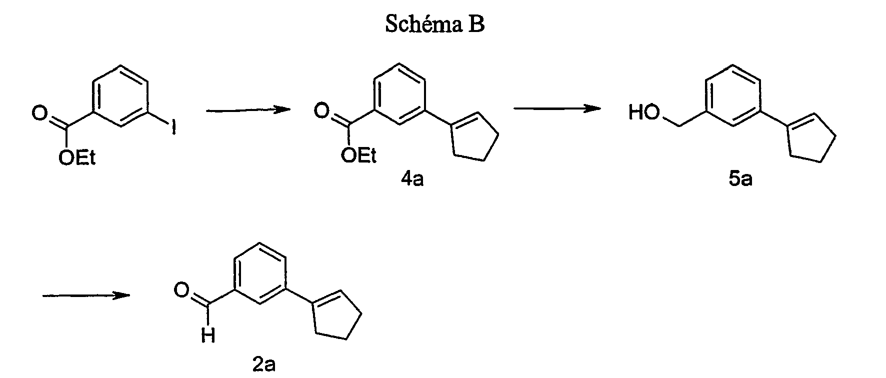

### Schéma B

Le 3-cyclopenten-1-yl-benzoate d'éthyle de formule (4a) est obtenu directement à partir du 3-iodobenzoate d'éthyle et du cyclopentène, disponibles commercialement, au moyen d'une réaction de Heck catalysée par du tris(dibenzylidèneacétone)dipalladium (RN 52409-22-0). La réduction de la fonction ester du composé de formule (4a) au moyen d'un agent donneur d'hydrure tel que, par exemple, de l'hydrure de lithium aluminium, conduit à l'alcool de formule (5 a). L'oxydation de la fonction alcool primaire en l'aldéhyde attendu de formule (2a) est ensuite effectuée au moyen de dioxyde de manganèse dans le chloroforme à chaud.

La préparation de l'aldéhyde (2b) dans lequel **X** représente un groupe (CH) et **Y** représente un atome de fluor, est décrite dans le schéma C.

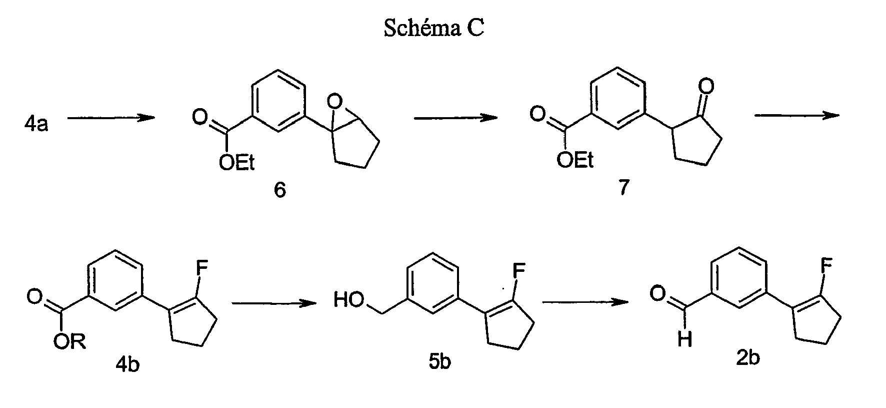

### Schéma C

Le 3-(2-oxo-cyclopentyl)-benzoate d'éthyle de formule (7) est obtenu par réarrangement (J. Org. Chem 1996, 61(5), 1877-79 et Synth. Commun. 1990, 20(12), 1751-56) de l'époxyde de formule (6), lui-même préparé par époxydation de la double liaison de l'intermédiaire (4a, schéma B) au moyen d'un peracide organique tel que, par exemple, l'acide m-chloroperbenzoique. La conversion de la fonction cétone du composé de formule (7) en une fonction gem-difluoro suivie de l'élimination de HF en milieu basique (Tetrahedron 1990, 46(12), 4255-60) donne le dérivé de formule (4b). La réduction de la fonction ester du composé (4b) en alcool primaire puis son oxydation selon une séquence analogue à celle décrite précédemment (cf. schéma B), conduit à l'aldéhyde attendu de formule (2b).

La préparation de l'aldéhyde (2c) dans laquelle **X** représente un atome d'azote et **Y** est un atome d'hydrogène est décrite dans le schéma D.

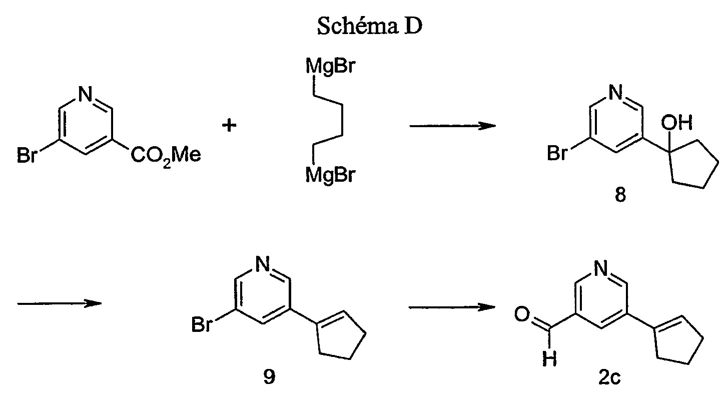

### Schéma D

L'addition du 1,4-bis(bromomagnesio)butane (RN 23708-47-6) sur l'ester méthylique de l'acide 5-bromo-nicotinique (RN 29681-44-5), effectuée selon un protocole analogue à celui décrit dans Eur. J. Med. Chem. 1991, 26, 563, conduit au 1-(5-bromo-pyridin-3-yl)-cyclopentanol de formule (8). Une réaction de déshydratation donne le dérivé insaturé (9) qui peut être ensuite converti en l'aldéhyde attendu (2c) en appliquant une méthode analogue à celle décrite dans Tetrahedron Lett. 2002, 43, 4285-87.

Les amines primaires de formule (3), dans lesquelles **W** et **(a)** sont tel que définis précédemment, peuvent être préparées selon une méthode analogue à celles décrites dans les brevets US 6,121,307 ; WO 0058282 et WO 0032557 (schéma E) : 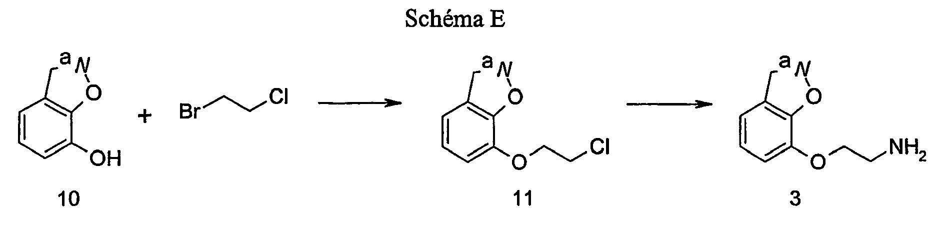 ainsi, la mono-alkylation d'un dérivé hydroxylé (10) convenablement substitué, au moyen du 1-bromo-2-chloro-éthane, disponible commercialement, conduit à l'éther de formule (11). L'atome d'azote est ensuite introduit par substitution de l'atome chlore du composé (11) au moyen d'un réactif approprié tel que, par exemple, de l'azidure de sodium ou du phtalimide de potassium. La fonction amine primaire est ensuite libérée soit par réduction de la fonction azido soit par hydrazinolyse de la fonction phtalimido pour donner les amines primaires correspondantes (3).

Les dérivés hydroxylés de formule (10), utilisés comme matières premières dans la synthèse des composés de formule (11), sont obtenus de la manière suivante :
- le composé (10a) dans lequel **W** est un groupe C(CH₃)₂ et **(a)** représente une liaison simple est disponible commercialement (RN 1563-38-8) ;
- le composé (10b) dans lequel **W** est un groupe CHCH₃ et **(a)** représente une liaison simple est décrit dans les brevets US 3,547,955 ; WO 8700840 et WO 9630367 ;
- le composé (10c) dans lequel **W** est un groupe CH et **(a)** représente une liaison double est préparé selon le brevet US 6,121,307 ;
- le composé (10d) dans lequel **W** est un groupe CCH₃ et **(a)** représente une liaison double est préparé selon la méthode décrite dans Tetrahedron 1996, 52(28), 9499-9508 ;
- le composé (10e) dans lequel **W** est un groupe CH₂ et **(a)** représente une liaison simple est préparé selon le brevet français N° de dépôt : 01 03877 ;
- le composé (10f) dans lequel **W** est un groupe C(CH₂)₂ et **(a)** représente une liaison simple est préparé selon le procédé illustré dans le schéma F, ci-après.

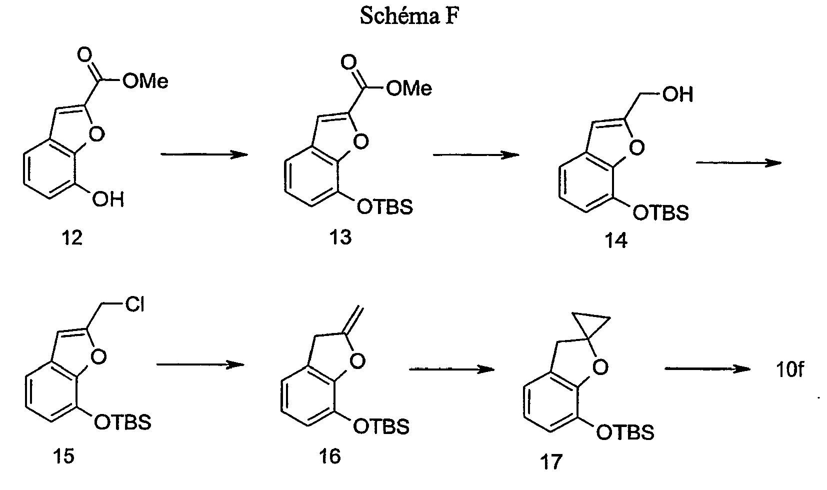

### Schéma F

Le groupe hydroxyle du composé de formule (12), EP 50957, est d'abord protégé sous la forme d'un éther silylé (dans le schéma F, l'abréviation TBS signifie tert-butyl-diméthylsilyl). La fonction ester du composé de formule (13) peut être alors réduite en alcool primaire au moyen d'un agent donneur d'hydrure (J. Pharm. Pharmacol. 1999, 51(4), 427-34). Après conversion du groupe hydroxyle du composé (14) en un atome de chlore, une réaction de réarrangement réductif (Tetrahedron Lett. 2001, 42, 939-41) permet d'accéder au dérivé exo-méthylène (16). Une réaction de cyclopropanation, effectuée selon J. Org. Chem. 1992, 57(19), 5271-76, fournit le composé spiro-propanique (17) qui est ensuite déprotégé pour donner le composé attendu (10f).

L'invention a aussi pour objet les compositions pharmaceutiques contenant à titre de principe actif au moins un des dérivés de formule générale (1) ou un de ses sels ou hydrates de ses sels en combinaison avec un ou plusieurs support inertes ou autres véhicules pharmaceutiquement acceptables.

Les compositions pharmaceutiques selon l'invention peuvent être, à titre d'exemple, des compositions administrables par voie orale, nasale, sublinguale, rectale ou parentérale.

A titre d'exemple de compositions administrables par voie orale on peut citer les comprimés, les gélules, les granules, les poudres et les solutions ou suspensions orales.

Les formulations appropriées pour la forme d'administration choisie sont connues et décrites, par exemple dans : Remington, The Science and Practice of Pharmacy, 19^{ième} édition, 1995, Mack Publishing Company.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter. En conséquence, la posologie optimale devra être déterminée, en fonction des paramètres jugés pertinents, par le spécialiste en la matière. Bien que les doses efficaces d'un composé de l'invention puissent varier dans de larges proportions, les doses journalières pourraient s'échelonner entre 0.001 mg et 100 mg par Kg de poids corporel de l'individu à traiter. Une dose journalière d'un composé de l'invention comprise entre 0.010 mg et 50 mg par Kg de poids corporel de l'individu à traiter étant, toutefois, préférée.

Les compositions pharmaceutiques selon l'invention sont utiles dans le traitement des psychoses schizophréniques.

### Exemples

Les exemples suivants illustrent l'invention sans toutefois en limiter la portée.

Dans les exemples ci-après :
(i) l'avancement des réactions est suivi par chromatographie couche mince (CCM) et par conséquent les temps de réaction ne sont mentionnés qu'à titre indicatif.
(ii) des formes cristallines différentes peuvent donner des points de fusions différents, les points de fusion rapportés dans la présente demande sont ceux des produits préparés selon la méthode décrite et ne sont pas corrigés.
(iii) la structure des produits obtenus selon l'invention est confirmée par les spectres de résonance magnétique nucléaire (RMN), infrarouge (IR) et l'analyse centésimale, la pureté des produits finaux est vérifiée par CCM.
(iv) les spectres RMN sont enregistrés dans le solvant indiqué. Les déplacements chimiques (δ) sont exprimés en partie par million (ppm) par rapport au tetraméthylsilane. La multiplicité des signaux est indiquée par : s, singulet ; d, doublet ; t, triplet ; q, quadruplet ; m, multiplet ; 1, large.
(v) les différents symboles des unités ont leur signification habituelle : mg (milligramme) ; g (gramme) ; ml (millilitre) ; °C (degré Celsius) ; mmole (millimole) ; nmole (nanomole) ; cm (centimètre).
(vi) Les abréviations ont la signification suivante : F (point de fusion) ; Eb (point d'ébullition).
(vii) Dans la présente application les pressions sont données en millibars ; par "température ambiante" on entend une température comprise entre 20°C et 25°C.

### Exemple 1 : 3-Cyclopenten-1-yl-benzoate d'éthyle (4a)

On introduit successivement dans un ballon : 7 g de 3-iodo benzoate d'éthyle (25 mmoles), 11.2 ml de cyclopentène (127 mmoles), 21 mL d'éthanol, 1.16 g de complexe Pd₂dba₃ (1.27 mmoles), 8.8 g de carbonate de potassium (63 mmoles) et 8.17 g de nBu₄NBr (25 mmoles). On chauffe à 80°C pendant 16 heures puis le mélange noir est filtré sur célite. Le précipité est lavé à l'acétate d'éthyle. Le filtrat est lavé à l'eau puis avec une solution aqueuse saturée de chlorure de sodium, séché sur sulfate de sodium, filtré puis concentré sous pression réduite. Le produit du titre, isolé par distillation au boule à boule (5.2 g), est obtenu sous la forme d'une huile jaune pâle.
¹H RMN (CDCl₃) : δ 1.39 (t, J = 7.1 Hz, 3H); 2.04 (m, 2H); 2.54 (m, 2H); 2.73 (m, 2H); 4.34 (q, J = 7.1 Hz, 2H); 6.27 (s, 1H); 7.35 (t, J = 7.7 Hz, 1H); 7.61 (d, J = 7.8 Hz, 1H); 7.88 (d, J = 7.8 Hz, 1H); 8.06 (s, 1H).

### Exemple 2 : (3-Cyclopenten-1-yl-phényl)-méthanol (5a)

A une suspension à 0°C de LiAlH₄ (0.57 g, 15 mmoles) dans l'éther éthylique (30 ml) on ajoute goutte à goutte une solution de 3-cyclopent-1-yl-benzoate d'éthyle (4a) (2.5 g, 12 mmoles) dans l'éther (25 ml). Le mélange est agité toute la nuit à température ambiante. Le mélange réactionnel est refroidi à 0°C puis on ajoute goutte à goutte 4.1 ml d'une solution aqueuse de soude à 10%. Le précipité blanc formé est filtré sous vide, le solide lavé avec de l'éther puis le filtrat concentré sous pression réduite. Le résidu est purifié par chromatographie flash sur gel de silice (cyclohexane / acétate d'éthyle : 90/10). Le produit du titre (1,35 g) est obtenu sous la forme d'une huile incolore.
¹H RMN (CDCl₃) : δ 2.02 (m, 2H); 2.53 (m, 2H); 2.72 (m, 2H); 3.74 (s, 1H); 4.68 (d, J = 6.0 Hz, 2H); 6.21 (s, 1H); 7.20 (d, J = 7.4 Hz, 1H); 7.29 (t, J = 7.6 Hz, 1H); 7.38 (d, J = 7.7 Hz, 1H); 7.44 (s, 1H).

### Exemple 3 : 3-Cyclopenten-1-yl-benzaldéhyde (2a)

Dans un ballon, on introduit 1.35 g (8 mmoles) de (3-cyclopenten-1-yl-phényl)-méthanol (5a) et 80 ml de chloroforme. On ajoute ensuite 6.8 g de MnO₂ et on chauffe la suspension à 60°C pendant 2 heures. Le mélange est filtré à chaud, le précipité lavé au chloroforme puis le filtrat concentré sous pression réduite. On obtient le produit du titre (1.05 g) sous la forme d'une huile jaune qui est utilisée dans l'étape suivante sans autre purification.
¹H RMN (CDCl₃) : δ 2.06 (m, 2H); 2.57 (m, 2H); 2.72 (m, 2H); 6.30 (s, 1H); 7.49 (t, J = 7.6 Hz, 1H); 7.71 (m, 2H); 7.91 (s, 1H); 10.02 (s, 1H).

### Exemple 4 : 3-(6-Oxa-bicyclo[3.1.0]hex-1-yl)-benzoate d'éthyle (6)

Dans un ballon, on introduit 5 g (23 mmoles) de 3-cyclopent-1-yl-benzoate d'éthyle (4a) et 100 ml de chlorure de méthylène. On refroidit la solution à 0°C et on ajoute par portions 9 g (28 mmoles) d'acide méta-chloroperbenzoïque. On agite le mélange pendant 30 minutes à 0°C puis 4 heures à température ambiante. Le mélange est filtré. Le filtrat est lavé successivement par une solution aqueuse saturée de thiosulfate de sodium, une solution aqueuse saturée de bicarbonate de sodium puis par une solution aqueuse saturée de chlorure de sodium. La phase organique est séchée sur Na₂SO₄, filtrée et concentrée sous pression réduite. Le résidu est purifié par chromatographie flash sur gel de silice (cyclohexane / acétate d'éthyle : 95/5). Le produit du titre est obtenu sous la forme d'une huile incolore (4.8 g).
¹H RMN (CDCl₃) : δ 1.40 (t, J = 7.2 Hz, 3H); 1.62 (m, 1H); 1.78 (m, 2H); 2.13 (m, 1H); 2.24 (m, 2H); 3.57 (s, 1H); 4.38 (q, J = 7.2 Hz, 2H); 7.41 (t, J = 7.7 Hz, 1H); 7.56 (d, J = 7.7 Hz, 1H); 7.96 (d, J = 7.7 Hz, 1H); 8.06 (s, 1H).

### Exemple 5 : 3-(2-Oxo-cyclopentyl)-benzoate d'éthyle (7)

Dans un ballon, on introduit 4 g (17.2 mmoles) de 3-(6-oxa-bicyclo[3.1.0]hex-1-yl)-benzoate d'éthyle (6) et 50 ml de chlorure de méthylène. Le mélange est refroidi à 0°C puis on ajoute goutte-à-goutte 2.2 ml (17.2 mmoles) de BF₃.Et₂O. On agite le mélange réactionnel à 0°C pendant 1 heure puis on ajoute 25 ml d'une solution aqueuse saturée de bicarbonate de sodium. Le mélange est décanté et la phase aqueuse extraite au chlorure de méthylène. Les phases organiques combinées sont séchées sur MgSO₄, filtrées et on concentrées sous pression réduite. Le résidu est purifié par chromatographie flash sur gel de silice (cyclohexane / acétate d'éthyle : 80/20). Le produit du titre est obtenu sous la forme d'une huile jaune pâle (3.4 g).
¹H RMN (CDCl₃) : δ 1.38 (t, J = 7.2 Hz, 3H); 1.98 (m, 1H); 2,17 (m, 2H); 2.31 (m, 1H); 2.51 (m, 2H); 3.38 (dd, J = 11.2; 8.6 Hz, 1H); 4.36 (q, J = 7.2 Hz, 2H); 7.39 (m, 2H); 7.86 (s, 1H); 7.93 (m, 1H).

### Exemple 6 : 3-(2-Fluoro-cyclopenten-1-yl)-benzoate d'alcoyle (4b)

Dans un ballon, on ajoute 4 g de 3-(2-oxo-cyclopentyl)-benzoate d'éthyle (7) (17.2 mmoles) et 8 ml de toluène. On ajoute ensuite goutte à goutte 9 ml de DAST (69 mmoles) et on porte le mélange à 60°C pendant 16 heures. On verse la solution dans un mélange glace / bicarbonate de sodium puis on extrait le mélange au chlorure de méthylène. La phase organique est lavée à l'eau, avec une solution aqueuse saturée de chlorure de sodium, séchée sur sulfate de sodium, filtrée puis concentrée sous pression réduite. Le résidu est purifié par filtration sur gel de silice (cyclohexane / acétate d'éthyle : 90/10). L'huile brune obtenu (2.9 g) est reprise dans du tétrahydrofuranne (50 ml) et la solution refroidie à -15°C. On ajoute lentement 34 ml de tert-butylate de potassium (1 M dans le tétrahydrofuranne, 34 mmoles) et on agite le mélange à -15°C pendant 3 heures. Le mélange est versé dans l'eau, extrait à l'éther puis les phases organiques combinées sont lavées avec une solution aqueuse saturée de chlorure de sodium, séchées sur MgSO₄ et concentrées sous pression réduite. On obtient une huile brune (2.5 g) utilisée dans l'étape suivante sans autre purification.

### Exemple 7 : 3-(2-Fluoro-cyclopenten-1-yl)-méthanol (5b)

On ajoute goutte à goutte une solution du dérivé (4b) (2 g, 9 mmoles) dans l'éther éthylique dans une suspension de LiAlH₄ (0.8 g, 21 mmoles) dans l'éther (20 ml) maintenue à 0°C. Le mélange réactionnel est lentement réchauffé à température ambiante puis agité pendant 16 heures. On ajoute ensuite à 0°C une solution aqueuse de soude à 10% (4 ml). Le précipité blanc formé est filtré sous vide, lavé à l'éther puis le filtrat concentré sous pression réduite. Le résidu est purifié par chromatographie flash sur gel de silice (cyclohexane / acétate d'éthyle : 70/30). On obtient le produit du titre sous la forme d'une huile jaune pâle (1.62 g).
¹H RMN (CDCl₃) : δ 1.58 (s, 1H); 2.02 (m, 2H); 2.70 (m, 4H); 4.70 (s, 2H); 7.23 (m, 1H); 7.34 (t, J = 7.7 Hz, 1H); 7.39 (m, 1H); 7.45 (m, 1H).

### Exemple 8 : 3-(2-Fluoro-cyclopenten-1-yl)-benzaldéhyde (2b)

Une suspension de MnO₂ (1.6 g) et 3-(2-fluoro-cyclopentèn-1-yl)-méthanol (5b) (0.64 g, 3.3 mmoles) dans 15 ml de chloroforme est chauffée à 60°C pendant 5 heures. On filtre le mélange à chaud, le précipité est lavé au chloroforme puis le filtrat concentré sous pression réduite. Le résidu est purifié par chromatographie flash sur gel de silice (cyclohexane / acétate d'éthyle : 80/20). Le produit du titre est obtenu sous la forme d'une et on obtient huile jaune (0.56 g).
¹H RMN (CDCl₃) : δ 2.04 (m, 2H); 2.74 (m, 4H); 7.51 (t, J = 7.7 Hz, 1H); 7.73 (d, J = 7.6 Hz, 1H); 7.93 (s, 1H); 7.79 (d, J = 7.7 Hz, 1H); 10.02 (s, 1H).

### Exemple 9 : 1-(5-Bromo-pyridin-3-yl)-cyclopentanol (8)

Dans un ballon contenant 2.25 g de copeaux de magnésium (92.6 mmoles) et un cristal d'iode on ajoute quelques gouttes de 1,4 dibromobutane et 25 ml de tétrahydrofurane. Le mélange est chauffé à 65°C jusqu'à décoloration puis on ajoute goutte à goutte une solution de 1,4 dibromobutane (10 g, 46.3 mmoles) dans 50 ml de tétrahydrofurane. Le mélange réactionnel est chauffé à 65°C pendant 4 heures puis refroidit à 0°C. On additionne alors une solution d'éthyl-2-bromo-nicotinate (10 g, 46.3 mmoles) dans 60 ml de tétrahydrofurane. Le mélange réactionnel est refroidi à température ambiante et agité pendant 16 heures. La réaction est versé lentement à 0°C dans une solution aqueuse saturée de chlorure d'ammonium puis on extrait à l'acétate d'éthyle. La phase organique est lavée à l'eau puis avec une solution aqueuse saturée de chlorure de sodium, séchée sur sulfate de sodium, filtrée et concentrée sous pression réduite. Le résidu est purifié par chromatographie flash sur gel de silice (cyclohexane / acétate d'éthyle : 70/30). Le produit du titre (3.6 g) est obtenu sous la forme d'un solide blanc.
F=74°C
¹H RMN (CDCl₃) : δ 1.76 (m, 2H); 1.87 (m, 6H); 2.73 (m, 2H); 3.93 (s, 1H); 7.15 (d, J = 5.0 Hz, 1H); 8.38 (d, J = 5.0 Hz, 1H); 8.64 (s, 1H).

### Exemple 10 : 3-Bromo-5-cyclopenten-1-yl-pyridine (9)

Une solution de 1-(5-bromo-pyridin-3-yl)-cyclopentanol (8) (1.7 g, 11.3 mmoles) dans 50 ml de toluène contenant 5 ml d'acide chlorhydrique concentré est chauffé à 120 °C pendant 12 heures avec entraînement de l'eau formée en continu. Le mélange est ensuite versé dans une solution aqueuse saturée de bicarbonate de sodium et extrait avec de l'acétate d'éthyle. Les phases organiques combinées sont lavées à l'eau puis avec une solution aqueuse saturée de chlorure de sodium, séchées sur sulfate de sodium, filtrées et concentrées sous pression réduite. Le résidu est purifié par chromatographie flash sur gel de silice (cyclohexane / acétate d'éthyle : 60/40). Le produit du titre est obtenu sous la forme d'un solide beige (1.65 g).
F = 43°C.
¹H RMN (CDCl₃) : δ 2.05 (m, 2H); 2.57 (m, 2H); 2.72 (m, 2H); 6.31 (s, 1H); 7.82 (m, 1H); 8.49 (d, J = 2.0 Hz, 1H); 8.59 (d, J = 2.0 Hz, 1H).

### Exemple 11 : 5-Cyclopenten-1-yl-pyridine-3-carboxaldéhyde (2c)

Une solution de 3-bromo-5-cyclopenten-1-yl-pyridine (9) (1 g, 4.5 mmoles) dans 25 ml d'éther est ajoutée à une solution de n-butyllithium (1.6 M dans l'hexane, 4.2 ml, 6.7 mmoles) dans 25 ml d'éther maintenu à -60°C. Le mélange réactionnel est agité pendant 2h30 à -60°C puis 1.4 ml de 4-morpholinecarboxaldehyde (13.4 mmoles) sont ajoutés. La réaction est agitée pendant 1 heure à -60°C puis versée dans l'eau, la phase organique est lavée avec une solution aqueuse saturée de chlorure de sodium, séchée sur sulfate de sodium, filtrée et concentrée sous pression réduite. Le résidu est purifié par chromatographie flash sur gel de silice (cyclohexane / acétate d'éthyle : 80/20). Le produit du titre est on obtenu sous la forme d'un solide beige (0.38 g).
F = 50°C.
¹H RMN (CDCl₃) : δ 2.10 (m, 2H); 2.61 (m, 2H); 2.72 (m, 2H); 6.49 (s, 1H); 8.32 (s, 1H); 8.89 (s, 1H); 8.93 (s, 1H); 10.10 (s, 1H).

### Exemple 12 : 7-(tert-Butyl-diméthyl-silanyloxy)-benzofuran-2-methoxycarbonyle (13)

On ajoute à une solution de 7-hydroxy-benzofuran-2-methoxycarbonyle (1.96 g, 10.2 mmoles) dans du diméthylformamide (10 ml), maintenue à 0°C, 1.61 g de chlorure de ter-butyl-diméthylsilane (10.7 mmoles) et 0.73 g d'imidazole (10.7 mmoles). Le mélange est agité pendant 16 heures à température ambiante. Le mélange est ensuite versé dans de l'eau, extrait avec de l'acétate d'éthyle et les phases organiques combinées sont lavées avec une solution aqueuse saturée de chlorure de sodium, séchées sur MgSO₄ et concentrées sous pression réduite. Le résidu est purifié par chromatographie flash sur gel de silice (cyclohexane / acétate d'éthyle : 95/5). On obtient le produit du titre sous la forme d'une huile jaune pâle (3.1 g).
¹H RMN (CDCl₃) : δ 0.27 (s, 6H); 1.05 (s, 9H); 3.95 (s, 3H); 6.92 (d, J = 7.7 Hz, 1H); 7.14 (t, J = 7.8 Hz, 1H); 7.25 (d, J = 7.8 Hz, 1H); 7.49 (s, 1H).

### Exemple 13 : [7-(tert-Butyl-diméthyl-silanyloxy)-benzofuran-2-yl]-methanol (14)

On ajoute goutte à goutte une solution de LiAlH₄ (8.4 ml, 8.4 mmoles) dans d'éther éthylique (1.0 M) à une solution de 7-(ter-butyl-diméthyl-silanyloxy)-benzofuran-2-méthoxycarbonyle (13) (2.15 g, 7 mmoles) dans l'éther (14 ml) maintenue à 0°C. Le mélange est agité pendant 18 heures à température ambiante puis refroidi à 0°C et traité goutte à goutte par une solution aqueuse de soude à 10% (1.6 ml). Le précipité formé est filtré sous vide et lavé à l'éther. Le filtrat est concentrée sous pression réduite pour donner un liquide incolore (1.8 g) utilisé dans l'étape suivante sans autre purification.
¹H RMN (CDCl₃) : δ 0.24 (s, 6H); 1.04 (s, 9H); 1.85 (t, J = 6.4 Hz, 1H); 4.76 (d, J = 6.0 Hz, 2H); 6.64 (s, 1H); 6.76 (d, J = 7.7 Hz, 1H); 7.04 (t, J = 7.7 Hz, 1H); 7.14 (d, J = 7.7 Hz, 1H).

### Exemple 14 : tert-Butyl-dimethyl-(2-chlorométhyl-benzofuran-7-yloxy)-silane (15)

On additionne à une solution de [7-(ter-butyl-diméthyl-silanyloxy)-benzofuran-2-yl]-méthanol (14) (1.78 g, 6.4 mmoles) dans du chlorure de méthylène (9 ml) maintenue à 0°C, 2.5 g (9.5 mmoles) de triphénylphosphine et 0.92 ml (9.5 mmoles) de tétrachlorure de carbone. Le mélange réactionnel est agité à 0°C pendant 1 heure puis le solvant est évaporé sous pression réduite. Le résidu est repris dans 30 ml de cyclohexane et agité pendant 1 heure. Le précipité formé est filtré et le filtrat concentré. Le résidu est purifié par chromatographie flash sur gel de silice (cyclohexane / acétate d'éthyle : 95/5). Le produit du titre est obtenu sous la forme d'un liquide jaune pâle (1.5g).
¹H RMN (CDCl₃) : δ 0.25 (s, 6H); 1.04 (s, 9H); 4.70 (s, 2H); 6.70 (s, 1H); 6.80 (d, J = 7.5 Hz, 1H); 7.07 (t, J = 7.7 Hz, 1H); 7.14 (d, J = 7.2 Hz, 1H).

### Exemple 15 : tert-Butyl-diméthyl-(2-méthylène-2,3-dihydro-benzofuran-7-yloxy)-silane (16)

LiAlH₄ (1.0 M dans le tétrahydrofuranne, 4.5 ml, 4.5 mmoles) est ajouté goutte à goutte à une suspension de CrCl₃ (1.4 g, 8.85 mmoles) dans du tétrahydrofuranne (10 ml) maintenue à 0°C et le mélange est agité pendant 15 minutes. La solution est ensuite diluée avec du diméthylformamide (18 ml) et de l'isopropanol (1.35 ml). A la solution obtenu maintenu à 0°C, on ajoute le ter-butyl-diméthyl-(2-chlorométhyl-benzofuran-7-yloxy)-silane (15) (1.05 g, 3.54 mmoles) dans du diméthylformamide (15 ml). Le mélange est agité à température ambiante pendant 18 heures puis versé dans de l'eau et extrait au pentane. La phase organique est séchée sur Na₂SO₄, filtrée et concentrée sous pression réduite. On obtient un liquide incolore (0,78 g) qui est utilisé dans l'étape suivante sans autre purification.
¹H RMN (CDCl₃) : δ 0.21 (s, 6H); 1.00 (s, 9H); 3.89 (s, 2H); 4.26 (d, J = 2.0 Hz, 1H); 4.69 (d, J = 2.0 Hz, 1H); 7.72 (m, 1H); 7.79 (m, 1H).

### Exemple 16 : tert-Butyl-diméthyl-(2-spirocyclopropane-2,3-dihydro-benzofuran-7-yloxy)-silane (17)

Une solution de diéthyl zinc dans le toluène (1.1 M, 6.8 ml, 7.48 mmol) est ajouté à une solution de ter-butyl-diméthyl-(2-méthylène-2,3-dihydro-benzofuran-7-yloxy)-silane (16) (0.78 g, 2.97 mmoles) dans du dichloroéthane (15 ml) maintenue à 0°C. Après la fin de l'addition, on ajoute 1.1 ml de chloroiodométhane (15 mmoles) et le mélange est agité à 0°C pendant 1 heure puis à 50°C pendant 1.5 heures. Le mélange est refroidit à 0°C et on additionne une solution aqueuse saturée de chlorure d'ammonium (10 ml). Après 15 minutes d'agitation le mélange est dilué avec du chlorure de méthylène, lavé avec de l'eau puis avec une solution aqueuse saturée de chlorure de sodium. La phase organique est séchée sur Na₂SO₄, filtrée et concentrée sous pression réduite. Le résidu est purifié par chromatographie flash sur gel de silice (cyclohexane / acétate d'éthyle : 98/2). Le produit du titre est obtenu sous la forme d'une huile incolore (0.75 g).
¹H RMN (CDCl₃) : δ 0.15 (s, 6H); 0.67 (s, 2H); 0.96 (s, 9H); 1.16 (s, 2H); 3.28 (s, 2H); 6.69 (m, 2H); 6.77 (m, 1H).

### Exemple 17 : 2-Spirocyclopropane-2,3-dihydro-benzofuran-7-ol (10f)

Une solution de fluorure de tétrabutylammonium (1.0 M dans le tétrahydrofuranne, 3.1 ml, 3.1 mmoles) est additionnée à une solution de ter-butyl-diméthyl-(2-spirocyclopropane-2,3-dihydro-benzofuran-7-yloxy)-silane (17) (0.57 g, 2.06 mmoles) dans 10 ml de tétrahydrofuranne à 0°C. La solution est agité à 0°C pendant 2 heures puis versée dans de l'eau et extraite à l'acétate d'éthyle. La phase organique est lavée avec une solution aqueuse saturée de chlorure de sodium, séchée sur Na₂SO₄, filtrée et concentrée sous pression réduite. Le résidu est purifié par chromatographie flash sur gel de silice (cyclohexane / acétate d'éthyle : 80/20). On obtient produit du titre (0.3 g) sous la forme d'un solide blanc.
F = 85-86°C ;
¹H RMN (CDCl₃) : δ 0.71 (t, J = 6.4 Hz, 2H); 1.20 (t, J = 6.4 Hz, 2H); 3.34 (s, 2H); 4.77 (s, 1H); 6.76 (m, 3H).

### Exemple 18 : [2-(2,2-Diméthyl-2,3-dihydro-benzofuran-7-yloxy)-éthyl]-(3-cyclopenten-1-yl-benzyl)-amine (1a)

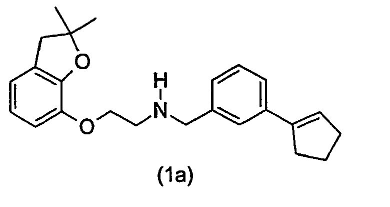

On ajoute 1.5 g de sulfate de magnésium dans une solution de 3-cyclopenten-1-yl-benzaldéhyde (2a) (0.56 g, 3.26 mmoles) et de [2-(2,2-diméthyl-2,3-dihydrobenzofuran-7-yloxy)]-éthylamine (3a) (0.68 g, 3.26 mmoles) dans 15 ml de 1,2-dichloroéthane et on chauffe le mélange à 60°C pendant 17 heures. Le mélange est refroidi à température ambiante, le solide est filtré et le solvant est évaporé sous pression réduite. On dilue le résidu avec 15 ml de méthanol puis on refroidit à 0°C. On introduit alors 0.35 g de borohydrure de potassium (6.52 mmoles) et le mélange réactionnel est agité pendant trois heures à 0°C. Le mélange est ensuite versé dans de l'eau glacée, extrait avec de l'acétate d'éthyle et lavé avec une solution aqueuse saturée de chlorure de sodium. Les phases organiques combinées sont séchées sur sulfate de magnésium, filtrées et le solvant est évaporé sous pression réduite. Le résidu est purifié par chromatographie sur gel de silice (chlorure de méthylène / méthanol / ammoniaque : 98/1.5/0.5). On isole le produit du titre (0.61 g) sous la forme d'une huile incolore.
¹H RMN (CDCl₃) : δ 1.48 (s, 6H); 2.00 (m, 2H); 2.54 (m, 2H); 2.69 (m, 2H); 3.01 (s, 2H); 3.04 (t, J = 5.6 Hz, 2H); 3.85 (s, 2H); 4.18 (t, J = 5.6 Hz, 2H); 6.18 (s, 1H); 6.74 (m, 3H); 7.19 (d, J = 7.4 Hz, 1H); 7.25 (t, J = 8.7 Hz, 1H); 7.32 (d, J = 7.6 Hz, 1H); 7.41 (s, 1H).
Fumarate du composé du titre :
F =146°C
¹H RMN (DMSOd⁶) : δ 1.39 (s, 6H); 1.96 (m, 2H); 2.51 (m, 2H); 2.65 (m, 2H); 2.96 (t, J = 5.6 Hz, 2H); 2.99 (s, 2H); 3.91 (s, 2H); 4.11 (t, J = 5.6 Hz, 2H); 6.27 (s, 1H); 6.56 (s, 2H); 6.71 (m, 1H); 6.79 (m, 2H); 7.31 (m, 2H); 7.37 (d, J = 7.6 Hz, 1H); 7.50 (s, 1H);
IR (KBr) ν : 3060, 2967, 1719, 1463 cm⁻¹;

| | | | |
|---|---|---|---|
| Analyse Elémentaire pour C₂₄H₂₉NO₂.C₄H₄O₄ | | | |
| théorique % : | C 70,13 | H 6,94 | N 2,92 |
| trouvé : | C 69,92 | H 6,93 | N 2,89. |

### Exemple 19 : [2-(Benzofuran-7-yloxy)-éthyl]-(3-cyclopenten-1-yl-benzyl)-amine (1c)

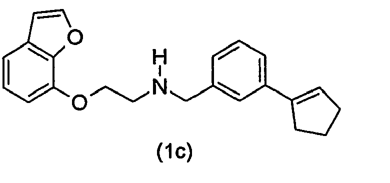

En procédant comme dans l'exemple 18 mais en utilisant la 2-(benzofuran-7-yloxy)éthyl-amine de formule (3c) à la place de la de [2-(2,2-diméthyl-2,3-dihydrobenzofuran-7-yloxy)]-éthylamine de formule (3a) on obtient le composé du titre.
¹H RMN (CDCl₃) : δ 2.04 (m, 2H); 2.53 (m, 2H); 2.70 (m, 2H); 3.12 (t, J = 5.2 Hz, 2H); 3.90 (s, 2H); 4.33 (t, J = 5.2 Hz, 2H); 6.19 (s, 1H); 6.76 (s, 1H); 6.83 (d, J = 7.6 Hz, 1H); 7.13 (t, J = 7.8 Hz, 1H); 7.19 (m, 2H); 7.29 (m, 2H); 7.33 (d, J = 7.5 Hz, 1H); 7.44 (s, 1H); 7.61 (d, J = 2.0 Hz, 1H).
Fumarate du produit du titre :
F =126°C
¹H RMN (DMSOd⁶) : δ 1.95 (m, 2H); 2.49 (m, 2H); 2.64 (m, 2H); 3.04 (t, J = 5.6 Hz, 2H); 3.91 (s, 2H); 4.29 (t, J = 5.6 Hz, 2H); 6.26 (s, 1H); 6.57 (s, 2H); 6.93 (m, 2H); 7.15 (t, J = 7.8 Hz, 1H); 7.26 (m, 3H); 7.36 (d, J = 7.2 Hz, 1H); 7.49 (s, 1H); 7.95 (s, 1H);
IR (KBr) ν : 3498, 2952, 2842, 1701, 1486 cm⁻¹;

| | | | |
|---|---|---|---|
| Analyse Elémentaire pour C₂₂H₂₃NO₂.C₄H₄O₄ | | | |
| théorique % : | C 69,47 | H 6,05 | N 3,12 |
| trouvé : | C 69,25 | H 6,08 | N 3,05. |

### Exemple 20 : [2-(2-Méthyl-benzofuran-7-yloxy)-éthyl]-(3-cyclopenten-1-yl-benzyl)-amine (1d)

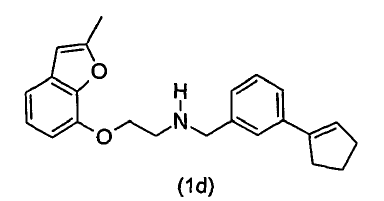

En procédant comme dans l'exemple 18 mais en utilisant la 2-(2-méthylbenzofuran-7-yloxy)-éthyl-amine de formule (3d) à la place de la de [2-(2,2-diméthyl-2,3-dihydro-benzofuran-7-yloxy)]-éthylamine de formule (3a) on obtient le composé du titre.
¹H RMN (CDCl₃) : δ 2.01 (m, 2H); 2.45 (s, 3H); 2.53 (m, 2H); 2.71 (m, 2H); 3.12 (t, J = 5.6 Hz, 2H); 3.89 (s, 2H); 4.32 (t, J = 5.6 Hz, 2H); 6.20 (s, 1H); 6.36 (s, 1H); 6.76 (m, 1H); 7.07 (m, 2H); 7.21 (d, J = 7,4 Hz, 1H); 7.28 (m, 2H); 7.33 (d, J = 7,6 Hz, 1H ); 7.43 (s, 1H).
Fumarate du produit du titre :
F =133°C
¹H RMN (DMSOd⁶) : δ 1.96 (m, 2H); 2.43 (s, 3H); 2.49 (m, 2H); 2.67 (m, 2H); 3.02 (t, J = 5.6 Hz, 2H); 3.89 (s, 2H); 4.26 (t, J = 5.6 Hz, 2H); 6.26 (s, 1H); 6.55 (s, 1H); 6.57 (s, 2H); 6.84 (m, 1H); 7.08 (m, 2H); 7.28 (m, 2H); 7.35 (d, J = 7.3 Hz, 1H); 7.49 (s, 1H);
IR (KBr) ν : 3421, 3048,2952,2846,1709,1587 cm⁻¹;

| | | | |
|---|---|---|---|
| Analyse Elémentaire pour C₂₃H₂₅NO₂.C₄H₄O₄ | | | |
| théorique % : | C 69,96 | H 6,31 | N 3,02 |
| trouvé : | C 70,04 | H 6,30 | N 2,98. |

### Exemple 21 : [2-(2,3-Dihydro-benzofuran-7-yloxy)-éthyl]-(3-cyclopenten-1-yl-benzyl)-amine (1e)

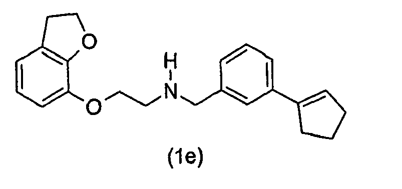

En procédant comme dans l'exemple 18 mais en utilisant la 2-(2,3-dihydro-benzofuran-7-yloxy)-éthyl-amine de formule (3e) à la place de la de [2-(2,2-diméthyl-2,3-dihydrobenzofuran-7-yloxy)]-éthylamine de formule (3a) on obtient le composé du titre.
¹H RMN (DMSOd⁶) : δ 1.95 (m, 2H); 2.40 (m, 2H); 2.65 (m, 2H); 2.81 (t, J = 5.6 Hz, 2H); 3.13 (t, J = 8.8 Hz, 2H); 3.74 (s, 2H); 4.03 (t, J = 5.6 Hz, 2H); 4.46 (t, J = 8.8 Hz, 2H); 6.25 (s, 1H); 6.79 (m, 3H); 7.27 (m, 3H); 7.42 (s, 1H).
Fumarate du produit du titre :
F = 118°C
¹H RMN (DMSOd⁶) : δ 1.92 (m, 2H); 2.49 (m, 2H); 2.65 (m, 2H); 2.93 (t, J = 5.6 Hz, 2H); 3.16 (t, J = 8.8 Hz, 2H); 3.88 (s, 2H); 4.11 (t, J = 5.6 Hz, 2H); 4.50 (t, J = 8.8 Hz, 2H); 6.27 (s, 1H); 6.56 (s, 2H); 6.81 (m, 3H); 7.24 (d, J = 6.9 Hz, 1H); 7.30 (t, J = 7.4 Hz); 7.36 ( d, J = 7.3 Hz, 1H); 7.48 (s, 1H);
IR (KBr) v : 3536, 3448, 2949, 2851, 1612, 1466 cm⁻¹;

| | | | |
|---|---|---|---|
| Analyse Elémentaire pour C₂₂H₂₅NO₂.C₄H₄O₄ | | | |
| théorique % : | C 69,16 | H 6,47 | N 3,10 |
| trouvé : | C 68,99 | H 6,55 | N 3,32. |

### Exemple 22 : [2-(2-spiro-cyclopropyl-2,3-dihydro-benzofuran-7-yloxy)-éthyl]-(3-cyclopenten-1-yl-benzyl)-amine (1f)

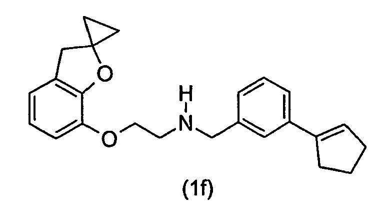

En procédant comme dans l'exemple 18 mais en utilisant la 2-(2-spiro-cyclopropyl-2,3-dihydro-benzofuran-7-yloxy)-éthyl-amine de formule (3f) à la place de la de [2-(2,2-diméthyl-2,3-dihydro-benzofuran-7-yloxy)]-éthylamine de formule (3a) on obtient le composé du titre.
¹H RMN (CDCl₃) : δ 0.69 (t, J = 6.4 Hz, 2H); 1.22 (t, J = 6.4 Hz, 2H); 1.23 (s, 1H); 2.01 (m, 2H); 2.51 (m, 2H); 2.70 (m, 2H); 3.01 (t, J = 5.2 Hz, 2H): 3.31 (s, 2H); 3.84 (s, 2H); 4.17 (t, J = 5.2 Hz, 2H); 6.18 (s, 1H); 6.83 (m, 3H); 7.18 (d, J = 7.4 Hz, 1H); 7.25 (t, J = 7.6 Hz, 1H); 7.31 (d, J = 7.6 Hz, 1H); 7.40 (s, 1H).
Maléate du produit du titre :
F =180°C
¹H RMN (DMSOd⁶) : δ 0.78 (t, J = 6.4 Hz, 2H); 1.06 (t, J = 6.4 Hz, 2H); 1.98 (m, 2H); 2.51 (m, 2H); 2.67 (m, 2H); 3.32 (m, 4H); 4.23 (m, 4H); 6.02 (s, 2H); 6.32 (s, 1H); 6.83 (m, 3H); 7.38 (m, 2H); 7.51 (d, J = 7.6 Hz, 1H); 7.60 (s, 1H);
IR (KBr) ν : 3454, 2998, 2957, 2841, 1621, 1461 cm⁻¹;

| | | | |
|---|---|---|---|
| Analyse Elémentaire pour C₂₄H₂₇NO₂.C₄H₄O₄ | | | |
| théorique % : | C 70,42 | H 6,54 | N 2,93 |
| trouvé : | C 70,27 | H 6,59 | N 3,14. |

### Exemple 23 : [2-(2,2-Diméthyl-2,3-dihydro-benzofuran-7-yloxy)-éthyl]-[3-(2-fluorocyclopenten-1-yl)-benzyl]-amine (1g)

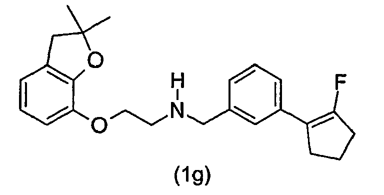

En procédant comme dans l'exemple 18 mais en utilisant le 3-(2-fluoro-cyclopenten-1-yl)-benzaldéhyde de formule (2b) à la place du 3-cyclopenten-1-yl-benzaldéhyde de formule (2a) on obtient le composé du titre.
¹H RMN (CDCl₃) : δ 1.48 (s, 6H); 1.97 (s, 1H); 2.01 (m, 2H); 2.68 (m, 4H); 3.00 (s, 2H); 3.03 (t, J = 5.2 Hz, 2H); 3.86 (s, 2H); 4.19 (t, J = 5.2 Hz, 2H); 6.78 (m, 3H); 7.21 (d, J = 7.5 Hz, 1H); 7.29 (t, J = 7.6 Hz, 1H); 7.41 (d, J = 7.6 Hz, 1H); 7.44 (s, 1H).
Fumarate du produit du titre :
F=145°C
¹H RMN (DMSOd⁶) : δ 1.39 (s, 6H); 1.95 (m, 2H); 2.67 (m, 4H); 2.94 (t, J = 5.6 Hz, 2H); 3.03 (s, 2H); 3.87 (s, 2H); 4.09 (t, J = 5.6 Hz, 2H); 6.57 (s, 2H); 6.72 (m, 1H); 6.79 (m, 2H); 7.27 (d, J = 7.5 Hz, 1H); 7.34 (m, 2H); 7.47 (s, 1H);
IR (KBr) v : 3426, 2962, 1675, 1463 cm⁻¹;

| | | | |
|---|---|---|---|
| Analyse Elémentaire pour C₂₄H₂₈NFO₂.C₄H₄O₄ | | | |
| théorique % : | C 67,59 | H 6,48 | N 2,82 |
| trouvé : | C 67,44 | H 6,59 | N 2,88. |

### Exemple 24 : [2-(2,2-Diméthyl-2,3-dihydro-benzofuran-7-yloxy)-éthyl]-(5-cyclopenten-1-yl-pyridin-3-ylméthyl)-amine (1b)

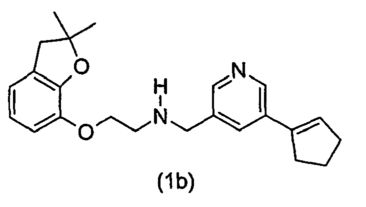

En procédant comme dans l'exemple 18 mais en utilisant le 5-cyclopenten-1-yl-pyridin-3-yl-carboxaldéhyde de formule (2c) à la place du 3-cyclopenten-1-yl-benzaldéhyde de formule (2a) on obtient le composé du titre.
¹H RMN (CDCl₃) : δ 1.74 (s, 6H); 2.06 (m, 2H); 2.54 (m, 2H); 2.70 (m, 2H); 3.01 (m, 4H); 3.86 (s, 2H); 4.19 (t, J = 5.2 Hz, 2H); 6.28 (s, 1H); 6.75 (m, 3H); 7.71 (s, 1H); 8.49 (s, 1H); 8.57 (s, 1H).
Fumarate du produit du titre :
F =141°C
¹H RMN (DMSOd⁶) : δ 1.39 (s, 6H); 1.98 (m, 2H); 2.51 (m, 2H); 2.69 (m, 2H); 2.92 (t, J = 5.6 Hz, 2H); 2.98 (s, 2H); 3.89 (s, 2H); 4.08 (t, J = 5.6 Hz, 2H); 6.42 (s, 1H); 6.59 (s, 2H); 6,71 (m, 1H); 6,78 (m, 2H); 7.84 (s, 1H); 8.50 (s, 1H); 8.59 (s, 1H);
IR (KBr) ν : 3036, 2973, 2847, 1715, 1618, 1491 cm⁻¹;

| | | | |
|---|---|---|---|
| Analyse Elémentaire pour C₂₃H₂₈N₂O₂.C₄H₄O₄ | | | |
| théorique % : | C 67.48 | H 6.71 | N 5.83 |
| trouvé : | C 67.14 | H 6.72 | N 5.79. |

Etude pharmacologique des composés de l'invention.

### 1- Mesure de l'affinité des composés de l'invention pour les récepteurs D₂.

L'affinité in vitro des composés de l'invention pour les récepteurs du type D₂ a été déterminé par la mesure du déplacement du (³H) YM-09151-2 (NET-1004 70-87 Ci / mmol), selon la méthode décrite dans Naunyn-Schimiedeberg's Arch. Pharmacol. Methods, 1985, 329, 333. Les valeurs de pKi, (pKi = -log Ki), sont données sous forme de moyenne ± SEM d'au moins 3 expérimentations.

### 2- Mesure de l'affinité des composés de l'invention pour les récepteurs 5-HT1_{A}.

L'affinité in vitro des composés de l'invention pour les récepteurs du sous-type 5-HT_{1A} a été déterminée par la mesure du déplacement de la [³H]8-OH-DPAT (TRK 850 ; 160-240 Ci/mmole). L'étude de la liaison au récepteur 5-HT_{1A} est réalisée comme décrit par Sleight et Peroutka (Naunyn-Schmiedeberg's Arch. Pharmaco. 1991, 343, 106).

Les valeurs de pKi, (pKi = -log Ki), sont données sous forme de moyenne ± SEM d'au moins 3 expérimentations.

### 3- Evaluation de l'activité antagoniste des récepteurs D₂ in vivo.

Le test mettant en évidence l'activité anti-dopaminergique in vivo des composés de l'invention repose sur l'inhibition des comportements induit par le méthylphénidate, mesuré chez le rat, selon la méthode décrite dans J. Pharmacol. Exp. Ther. 1993, 267, 181.

### 4- Evaluation des effets cataleptigènes des composés de l'invention.

Le test permettant d'évaluer la propension des produits de l'invention à provoquer des effets secondaires d'ordre extra-pyramidaux repose sur leur pouvoir cataleptigène, mesuré chez le rat, selon la méthode décrite dans Eur. J. Pharmacol. 1996, 313, 25.

Le tableau ci-dessous donne, à titre d'exemple, les valeurs des pKi, mesurées au niveau des récepteurs D₂ et 5-HT1_{A}, ainsi que les doses efficaces (DE₅₀) obtenues après administration de certains produits de l'invention par voie orale chez l'animal. Les propriétés des composés de l'invention sont comparées avec celles de substances choisies comme référence utilisées en clinique humaine i.e., le némonapride (composé mixte : antagoniste D₂ et agoniste 5-HT1_{A}), la risperidone (antipsychotique atypique) et le haloperidol (antipsychotique conventionnel).

**Tableau**

| Composé Composé | D₂ pKi | 5-HT1_{A} pKi | normalisation ED₅₀ mg / kg | Catalepsie ED₅₀ mg / kg |
|---|---|---|---|---|
| 1a | 9.5 | 8.2 | 1.3 | > 40 |
| 1f | 9.2 | 8.2 | 0.63 | > 40 |
| némonapride | 9.9 | 8.4 | 1.5 | 5.0 |
| Rispéridone | 8.7 | 6.0 | 6.5 | 3.5 |
| halopéridol | 9.0 | 5.8 | 0.46 | 0.92 |

Il ressort de cette étude que les composés de l'invention possèdent une affinité importante pour les récepteurs des sous-types D₂ et 5-HT1_{A}. Le rapport des valeurs de pKi, quasi-identique pour les composés de l'invention et le némonapride [pKi(D₂) / pKi(5-HT1_{A}) ≅ 1.3], montre que les composés de l'invention et le némonapride ont des profils d'affinité (D₂ et 5-HT1_{A}) comparables. La rispéridone et le halopéridol possèdent quand à eux une bonne affinité pour les récepteurs D₂ mais ne présentent qu'une faible affinité pour les récepteurs 5-HT1_{A}.

Les activités anti-dopaminergique in vivo des produits de l'invention et celles des composés de référence s'expriment dans des gammes de doses relativement comparables. Sur la base du critère de normalisation des stéréotypies, la contribution apportée par une activation 5-HT1_{A} n'apparaît donc pas de façon marquante. Cependant, nous constatons que parmi les composés de l'étude, les produits ayant une affinité importante pour les récepteurs 5-HT1_{A} (i.e., 1a, 1f et némonapride) présentent une moindre propension à provoquer de la catalepsie. Cette tendance est clairement illustré par la comparaison des rapports des doses cataleptigènes (effet indésirable) et celles nécessaires pour normaliser le comportement (activité pharmacologique recherchée); ainsi, ED₅₀(catalepsie) / ED₅₀(normalisation) > 1 dans le cas des produits 1a, 1f et du némonapride alors que ED₅₀(catalepsie) / ED₅₀(normalisation) < 1 dans le cas de la rispéridone et du halopéridol. Sur la base des résultats de catalepsie obtenus avec le némonapride (ED₅₀ = 5 mg/kg), il est surprenant que les composés de l'invention (i.e., 1a et 1f) soient eux dépourvus de tout effets cataleptigènes, même à de fortes doses (i.e., 40 mg/kg). Il est encore une fois avantageux de comparer les rapports ED₅₀(catalepsie) / ED₅₀(normalisation) ; ainsi, alors que ce rapport est environ égal à 3 dans le cas du némonapride, il devient supérieur à 30 pour les composés 1a et 1f. Il apparaît donc de façon évidente que :
- les activités anti-dopaminergique et sérotoninergique des composés de l'invention s'expriment toute les deux in vivo dans les gammes de doses testées ;
- les activités en question co-opèrent d'une manière telle que leur combinaison confère un avantage significatif aux composés de l'invention non seulement vis à vis des produits dont le mécanisme d'action est à priori similaire (e.g., némonapride) mais aussi vis à vis des antipsychotiques atypiques (e.g., rispéridone) et conventionnels (e.g., halopéridol).

Les composés de l'invention qui sont capables de se comporter comme des antagonistes dopaminergiques puissants et efficaces sans toutefois provoquer les effets secondaires caractéristiques des antagonistes dopaminergiques (i.e., catalepsie chez l'animal), et cela même à des doses très supérieures aux doses pharmacologiques, sont, de ce fait, potentiellement utiles dans le traitement des désordres dans lesquels un disfonctionnement dopaminergiques est impliqué, en particulier, les psychoses schizophréniques.

L'administration des composés de l'invention peut être réalisée par voie orale, nasale, sublinguale, rectale ou parentérale. A titre d'exemples de formulation non limitatifs, nous donnons ci-après, une préparation des composés de l'invention. Les ingrédients ainsi que d'autres, thérapeutiquement acceptables, peuvent être introduits en d'autres proportions sans modifier la portée de l'invention. Les termes 'ingrédient actif' utilisés dans l'exemple de formulation ci-après font références à un composé de formule (1) ou un sel d'addition ou éventuellement un hydrate d'un sel d'addition du composé de formule (1) avec un acide minéral ou un acide organique pharmaceutiquement acceptable.

### Exemple de composition pharmaceutique

Formule de préparation pour 1000 comprimés contenant chacun 10 mg de l'ingrédient actif :

| | |
|---|---|
| Ingrédient actif | 10 g |
| Lactose | 100 g |
| Amidon de blé | 10 g |
| Stéarate de magnésium | 3 g |
| Talc | 3 g |

## Revendications

1. Les composés de formule générale (1) 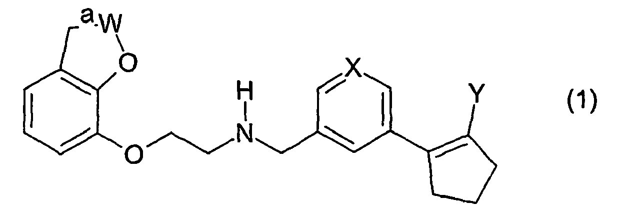 dans laquelle :
- **(a)** représente une liaison simple ou une liaison double ;
- **W** représente un groupe CH, CH₂, CHCH₃, CCH₃, C(CH₃)₂, un groupe C(CH₂)₂ (i.e., un atome de carbone portant deux groupes méthylènes liés entre eux de manière à former un motif spiro-cyclopropane) ou C(CH₂)₃ (i.e., un atome de carbone portant deux groupes méthylènes liés à un autre groupe méthylène de manière à former un motif spiro-cyclobutane) avec la réserve, toutefois, que lorsque **(a)** est une liaison double alors **W** représente exclusivement un groupe CH ou CCH₃ et que, lorsque **(a)** est une liaison simple, alors **W** représente exclusivement un groupe CH₂, CHCH₃, C(CH₃)₂, C(CH₂)₂ ou C(CH₂)₃ ;
- **X** est un atome de carbone portant un atome d'hydrogène (CH) ou un atome d'azote ;
- **Y** est un atome d'hydrogène ou un atome de fluor ;
leurs sels d'addition et éventuellement les hydrates des sels d'addition avec les acides minéraux ou les acides organiques pharmaceutiquement acceptables ainsi que leurs formes tautomères, les énantiomères purs et les mélanges d'énantiomères racémique ou non.

2. Dérivés selon la revendication 1, **caractérisé en ce qu'**ils sont choisis parmi les composés suivants :
[2-(2,2-Diméthyl-2,3-dihydro-benzofuran-7-yloxy)-éthyl]-(3-cyclopenten-1-yl-benzyl)-amine;
[2-(Benzofuran-7-yloxy)-éthyl]-(3-cyclopenten-1-yl-benzyl)-amine ;
[2-(2-Méthyl-benzofuran-7-yloxy)-éthyl]-(3-cyclopenten-1-yl-benzyl)-amine ;
[2-(2,3-Dihydro-benzofuran-7-yloxy)-éthyl]-(3-cyclopenten-1-yl-benzyl)-amine ;
[2-(2-Spiro-cyclopropyl-2,3-dihydro-benzofuran-7-yloxy)-éthyl]-(3-cyclopenten-1-yl-benzyl)-amine ;
[2-(2,2-Diméthyl-2,3-dihydro-benzofuran-7-yloxy)-éthyl]-[3-(2-fluoro-cyclopenten-1-yl)-benzyl]-amine ;
[2-(2,2-Diméthyl-2,3-dihydro-benzofuran-7-yloxy)-éthyl]-(5-cyclopenten-1-yl-pyridin-3-ylméthyl)-amine ;
leurs sels d'addition et éventuellement les hydrates des sels d'addition avec les acides minéraux ou les acides organiques pharmaceutiquement acceptables ainsi que leurs isomères et leurs tautomères.

3. Composés de formule (2) : 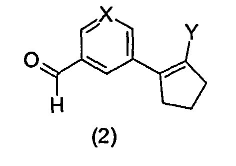 dans laquelle **X** et **Y** ont la même signification que dans la formule (1) comme intermédiaire de synthèse intervenant dans la préparation des composés de formule (1).

4. Composé selon l'une des revendications 1 et 2 à titre de médicaments.

5. Compositions pharmaceutiques **caractérisées en ce qu'**elles contiennent comme ingrédient actif au moins un composé selon l'une des revendications 1 et 2 associé à un support pharmaceutique inerte ou autres véhicules pharmaceutiquement acceptables et éventuellement à un autre médicament.

6. Compositions pharmaceutiques, selon la revendication 5 utiles dans le traitement de la schizophrénie ou utiles dans le traitement de l'évolution de la schizophrénie.

## Claims

1. Compounds of general formula (1) 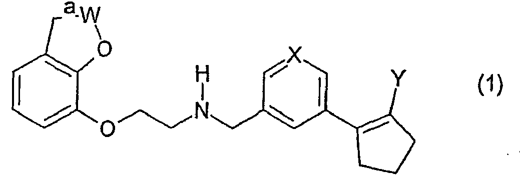 in which:
- **(a)** represents a single bond or a double bond;
- **W** represents a CH, CH₂, CHCH₃, CCH₃ or C(CH₃)₂ group, a C(CH₂)₂ group (i.e. a carbon atom bearing two methylene groups linked together so as to form a spiro-cyclopropane unit) or a C(CH₂)₃ group (i.e. a carbon atom bearing two methylene groups linked to another methylene group so as to form a spiro-cyclobutane unit) with the proviso, however, that when **(a)** is a double bond, then **W** exclusively represents a CH or CCH₃ group, and that when **(a)** is a single bond, then **W** exclusively represents a CH₂, CHCH₃, C(CH₃)₂, C(CH₂)₂ or C(CH₂)₃ group;
- **X** is a carbon atom bearing a hydrogen atom (CH) or a nitrogen atom;
- **Y** is a hydrogen atom or a fluorine atom;
their addition salts and optionally the hydrates of the addition salts with pharmaceutically acceptable inorganic acids or organic acids and their tautomeric forms, the pure enantiomers and mixtures of racemic or nonracemic enantiomers.

2. Derivatives according to Claim 1, **characterized in that** they are chosen from the following compounds:
[2-(2,2-dimethyl-2,3-dihydrobenzofuran-7-yloxy)ethyl]-(3-cyclopenten-1-ylbenzyl)amine;
[2-(benzofuran-7-yloxy)ethyl]-(3-cyclopenten-1-ylbenzyl)-amine;
[2-(2-methylbenzofuran-7-yloxy)ethyl]-(3-cyclopenten-1-ylberizyl)amine;
[2-(2,3-dihydrobenzofuran-7-yloxy)ethyl]-(3-cyclopenten-1-ylbenzyl)amine;
[2-(2-spirocyclopropyl-2,3-dihydrobenzofuran-7-yloxy)-ethyl]-(3-cyclopenten-1-ylbenzyl)amine;
[2-(2,2-dimethyl-2,3-dihydrobenzofuran-7-yloxy)ethyl]-[3-(2-fluorocyclopenten-1-yl)benzyl]amine;
[2-(2,2-dimethyl-2,3-dihydrobenzofuran-7-yloxy)ethyl]-(5-cyclopenten-1-ylpyridine-3-ylmethyl)amine;
their addition salts and optionally the hydrates of addition salts with pharmaceutically acceptable inorganic acids or organic acids and their isomers and their tautomers.

3. Compounds of formula (2): 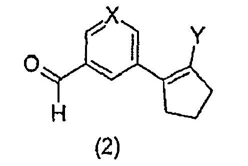 in which **X** and **Y** have the same meaning as in formula (1) as synthesis intermediate involved in the preparation of the compounds of formula (1).

4. Compound according to either of Claims 1 and 2, as medicaments.

5. Pharmaceutical compositions, **characterized in that** they contain, as active ingredient, at least one compound according to either of Claims 1 and 2 combined with an inert pharmaceutical carrier or other pharmaceutically acceptable vehicles and optionally with another medicament.

6. Pharmaceutical compositions according to Claim 5, which are useful in the treatment of schizophrenia or useful in the treatment of the progression of schizophrenia.

## Patentansprüche

1. Verbindungen der allgemeinen Formel (1) 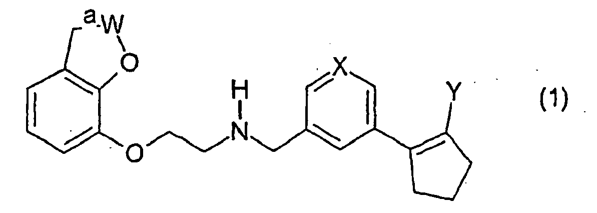 in der:
- (a) eine Einfachbindung oder eine Doppelbindung darstellt;
- W eine Gruppe CH, CH₂, CHCH₃, CCH₃, C(CH₃)₂, eine Gruppe C(CH₂)₂ (d.h. ein Kohlenstoffatom, das zwei Methylengruppen trägt, die untereinander auf solche Weise gebunden sind, dass sie eine Spirocyclopropan-Gruppe bilden) oder C(CH₂)₃ (d.h. ein Kohlenstoffatom, das zwei Methylengruppen trägt, die mit einer weiteren Methylengruppe auf solche Weise verbunden sind, dass sie eine Spirocyclobutan-Gruppe bilden) darstellt, jedoch mit der Maßgabe, dass, wenn (a) eine Doppelbindung ist, dann W ausschließlich eine Gruppe CH oder CCH₃ darstellt, und dass, wenn (a) eine Einfachbindung darstellt, dann W ausschließlich eine Gruppe CH₂, CHCH₃, C(CH₃)₂, C(CH₂)₂ oder C(CH₂)₃ darstellt;
- X ein Kohlenstoffatom ist, das ein Wasserstoffatom (CH) oder ein Stickstoffatom trägt;
- Y ein Wasserstoffatom oder ein Fluoratom ist;
deren Additionssalze und gegebenenfalls die Hydrate der Additionssalze mit pharmazeutisch annehmbaren Mineralsäuren oder organischen Säuren sowie deren tautomere Formen, die reinen Enantiomere und die racemischen oder nicht-racemischen Mischungen von Enantiomeren.

2. Derivate nach Anspruch 1, **dadurch gekennzeichnet, dass** sie aus den folgenden Verbindungen ausgewählt sind:
[2-(2,2-Dimethyl-2,3-dihydrobenzofuran-7-yloxy)ethyl]-(3-cyclopenten-1-ylbenzyl)amin;
[2-(Benzofuran-7-yloxy)ethyl]-(3-cyclopenten-1-ylbenzyl)amin;
[2-(2-Methylbenzofuran-7-yloxy)ethyl]-(3-cyclopenten-1-ylbenzyl)amin;
[2-(2,3-Dihydrobenzofuran-7-yloxy)ethyl]-(3-cyclopenten-1-ylbenzyl)amin;
[2-(2-Spirocyclopropyl-2,3-dihydrobenzofuran-7-yloxy)ethyl]-(3-cyclopenten-1-ylbenzyl)amin;
[2-(2,2-Dimethyl-2,3-dihydrobenzofuran-7-yloxy)ethyl]-[3-(2-fluorcyclopenten-1-yl)benzyl]amin;
[2-(2,2-Dimethyl-2,3-dihydrobenzofuran-7-yloxy)ethyl]-(5-cyclopenten-1-ylpyridin-3-ylmethyl)amin;
deren Additionssalze und gegebenenfalls die Hydrate der Additionssalze mit pharmazeutisch annehmbaren Mineralsäuren oder organischen Säuren sowie deren Isomere und deren Tautomere.

3. Verbindungen der Formel (2): 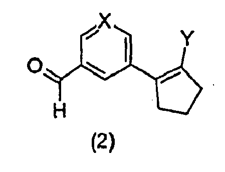 in der X und Y die gleiche Bedeutung wie in der Formel (1) aufweisen, als Synthesezwischenprodukt, das bei der Herstellung der Verbindungen der Formel (1) vorkommt.

4. Verbindung nach einem der Ansprüche 1 und 2 als Medikamente.

5. Pharmazeutische Zusammensetzungen, **dadurch gekennzeichnet, dass** sie als Wirkstoff mindestens eine Verbindung nach einem der Ansprüche 1 und 2 in Verbindung mit einem inerten pharmazeutischen Träger oder anderen pharmazeutisch annehmbaren Vehikeln und gegebenenfalls einem anderen Medikament enthalten.

6. Pharmazeutische Zusammensetzungen nach Anspruch 5, die bei der Behandlung von Schizophrenie nützlich sind oder bei der Behandlung der Entwicklung von Schizophrenie nützlich sind.
